# EUROPEAN PATENT APPLICATION

(11) **EP 4 470 445 A1**
(43) Date of publication of application: **04.12.2024**
(21) Application number: 22923379.6
(22) Date of filing: 04.11.2022
(51) Int. Cl.: A61B 1/00, A61B 1/04, A61B 1/06

(54) **FLUORESCENT ENDOSCOPE SYSTEM AND CONTROL METHOD THEREFOR**

(30) Priority: 27.01.2022 CN 202210102712
(71) Applicant: Scivita Medical Technology Co., Ltd., Suzhou, Jiangsu 215000 (CN)
(72) Inventor: CHIN, To, Suzhou, Jiangsu 215000 (CN)
(74) Representative: Nex & Phister Law & IP Aps
(86) International application number: PCT/CN2022/129766
(87) International publication number: WO 2023/142584

(57) **Abstract**

The present disclosure relates to a fluorescence endoscope system. The fluorescence endoscope system comprises a light source module, a camera module, a main control module and a display module. The light source module is configured to emit illumination beams. The camera module comprises an endoscope and an operation portion. The endoscope is configured to propagate the illumination beams and reflected beams thereof. The operation portion is configured to receive the reflected beams and convert the reflected beams into video data. The main control module comprises a control unit and an image processing unit. The control unit is configured to control the light source module to emit a first illumination beam or a second illumination beam. The image processing unit is configured to process the video data frame by frame. The display module is configured to play the video data. The present disclosure can provide a white-light mode and a fluorescence mode, and switch between the white-light mode and the fluorescence mode by a first button on an operation portion. A fluorescence image of a tissue under a tissue surface is provided by means of fluorescence imaging, such that inconspicuous lesions and early lesions can be visually and precisely identified, thereby improving the safety of surgery.

## Description

### TECHNICAL FIELD

The present disclosure relates to the technical field of a fluorescence endoscope, and in particular to a fluorescence endoscope system and a control method thereof.

### BACKGROUND

An endoscope system based on intrinsic optical spectral characteristics of human tissue is a novel medical device capable of entering the body for diagnostic and therapeutic purposes. It is designed to sensitively and specifically differentiate between benign and malignant lesions in the tissue, identifying abnormal tissues that are not visible under X-rays, thereby contributing to an increased detection rate of early-stage cancers. Fluorescence imaging, with its capability of visualizing and precisely identifying otherwise inconspicuous lesions and precancerous changes, holds significant advantages. Consequently, there is an urgent need to provide a fluorescence endoscopy system and its control method to ensure the safety of surgical procedures.

### SUMMARY

The technical problems to be solved by the present disclosure are to overcome the problems existing in the prior art, and thus the present disclosure provides a fluorescence endoscope system and a control method thereof.

The present disclosure provides a fluorescence endoscope system, comprising:
a light source module comprising a first light guide configured to guide illumination beams emitted by the light source module, wherein the illumination beams comprise a first illumination beam corresponding to a white-light mode and a second illumination beam corresponding to a fluorescence mode;
a camera module comprising an endoscope and an operation portion which are coupled to each other, wherein the endoscope is configured to propagate the illumination beam guided by the first light guide and the reflected beams formed after the illumination beam is irradiated to an object, and the operation portion is configured to receive the reflected beams and convert the reflected beams into video data;
a main control module comprising a control unit and an image processing unit, wherein the control unit is configured to control the light source module to emit the first illumination beam or the second illumination beam, and the image processing unit is configured to process the video data; and
a display module configured to play the video data processed by the image processing unit,
wherein the operation portion comprises a hand-held main body and a first button arranged on the hand-held main body, the operation portion is configured to generate a first control signal after the first button is triggered, the operation portion transmits the first control signal to the image processing unit, and the first control signal is capable of controlling the light source module to emit the first illumination beam or the second illumination beam to switch between the white-light mode and the fluorescence mode.

In one embodiment of the present disclosure, the first illumination beam is an illumination beam containing only a wide-spectrum white visible light having a wavelength of 400nm to 730nm, and the second illumination beam is an illumination beam mixing a white visible light and a narrow-band IR excitation light having a wavelength of 750nm to 810nm.

In one embodiment of the present disclosure, the camera module comprises an optical adapter through which the endoscope is coupled to the operation portion, and the optical adapter is configured to focus the reflected beams.

In one embodiment of the present disclosure, the endoscope comprises an objective lens, a light guide structure, an eyepiece, a light source interface, and a second light guide, the second light guide is arranged along the axial direction of the endoscope, the first light guide is coupled to the second light guide through the light source interface, the illumination beams encounter the object and changes a propagation direction to form the reflected beams, and the reflected beams pass through the objective lens, the light guide structure, the eyepiece and enter into the optical adapter.

In one embodiment of the present disclosure, the hand-held main body has a front end surface and a cavity, the front end surface is provided with an optical filter, a beam-splitting prism and an image sensor are arranged inside of the cavity, the optical adapter focuses the reflected beams on the filter On the optical sheet, the optical filter is configured to cut off the reflected beams with a wavelength greater than or equal to 750nm and less than or equal to 810nm, the beam-splitting prism is configured to split the reflected beams passing through the optical filter, and the image sensor is configured to receive the reflected beams split by the beam-splitting prism and convert the reflected beams to video data.

In one embodiment of the present disclosure, the operation portion is configured to being preset with a first mapping relationship between the first button and the first control signal, the first control signal is generated based on the first mapping relationship after the first button is triggered, and the first control signal is used to control the image processing unit to perform corresponding actions, wherein the first mapping relationship includes a first mapping relationship a and a first mapping relationship b corresponding to the white-light mode and the fluorescence mode respectively.

In one embodiment of the present disclosure, the main control module includes a panel assembly and a second button arranged on the panel assembly, the panel assembly is configured to generate a second control signal after the second button is triggered, and the panel assembly transmits the second control signal to the control unit, wherein the second control signal is used to control the light source module to emit the first illumination beam or the second illumination beam to switch between the white-light mode and the fluorescence mode.

In one embodiment of the present disclosure, the panel assembly is preset with the combination arrangement rule that the second button is triggered, and with a third mapping relationship between the combination arrangement rule and the second control signal, and after the second button is triggered according to the combination arrangement rule, the second control signal is generated based on the third mapping relationship to control the image processing unit to load a first GUI on the upper layer of the video data.

The present disclosure further provides a method for controlling the system mentioned above, comprising:
triggering the first button to control the light source module to emit the first illumination beam or the second illumination beam to switch between the white-light mode and the fluorescence mode; and/or
triggering the second button to control the light source module to emit the first illumination beam or the second illumination beam to switch between the white-light mode and the fluorescence mode.

In one embodiment of the present disclosure, further comprising: triggering the second button according to the combination arrangement rule, to control the image processing unit to load the first GUI on the upper layer of the video data.

The above technical solution of the present disclosure has the following advantages compared with the prior art.

The present disclosure can provide at least two modes including the white-light mode and the fluorescence mode, and switch between the white-light mode and the fluorescence mode by the first button on the operation portion. A fluorescence image of a tissue under a tissue surface is provided by means of fluorescence imaging, such that inconspicuous lesions and early lesions can be visually and precisely identified, thereby improving the safety of surgery.

### BRIEF DESCRIPTION OF THE DRAWINGS

In order to make the present disclosure more clearly understood, the present disclosure will be further described in detail below according to the specific embodiments of the present disclosure and in conjunction with the accompanying drawings.
Fig. 1 is a structural schematic diagram of the present disclosure.
Fig. 2 is a schematic structural diagram of the camera module of the present disclosure.
Fig. 3 is a schematic diagram of the structure of the operation portion of the present disclosure.
Fig. 4 is a schematic structural view of the panel assembly of the present disclosure.
Fig. 5 is a schematic diagram of an exemplary structure of an embodiment of the present disclosure.

Description of the reference numerals
100: light source module; 200: camera module; 210: endoscope; 211: objective lens; 212: light guide structure; 213: eyepiece; 214: light source interface; 220: operation portion; 221: hand-held main body; 222: optical filter; 223: beam-splitting prism; 224: image sensor; 225: first button; 230: optical adapter; 231: cavity; 232: lens; 300: main control module; 310: control unit; 320: image processing unit; 330: image cache unit; 340: external storage interface; 360: panel assembly; 361: second button; 400: display module.

### DETAILED DESCRIPTION

The present disclosure will be further described below in conjunction with the accompanying drawings and specific embodiments, so that those skilled in the art can better understand and implement the present disclosure. It should be noted that the embodiments provided are not intended to limit the present disclosure.

### EMBODIMENT ONE

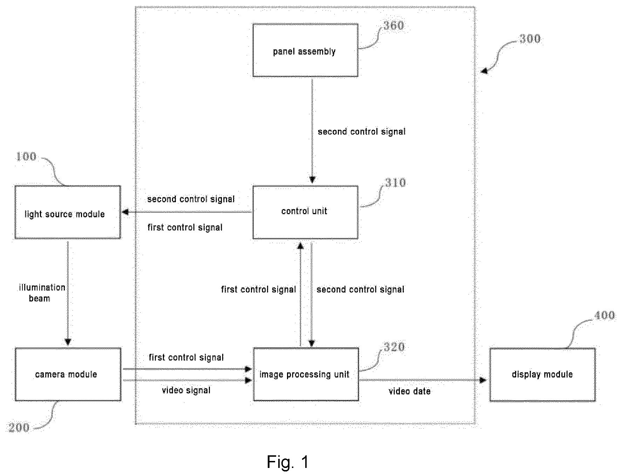

Referring to Figs. 1 to 4, the embodiment 1 of the present disclosure provides a fluorescence endoscope system. The fluorescence endoscope system includes a light source module 100, a camera module 200, a main control module 300, and a display module 400. The light source module 100 includes a first light guide (not shown in the figure). The first light guide is configured to guide illumination beams emitted by the light source module 100. The illumination beams include a first illumination beam and a second illumination beam corresponding to the white-light mode and the fluorescence mode respectively. The camera module 200 includes an endoscope 210 and an operation portion 220 which are coupled to each other. The endoscope 210 is configured to propagate the illumination beams and reflected beams formed after the illumination beams irradiate to an object. The operation portion 220 is configured to receive the reflected beams and convert the reflected beams into video data. The main control module 300 includes a control unit 310 and an image processing unit 320. The control unit 310 is configured to control the light source module 100 to emit the first illumination beam or the second illumination beam. The image processing unit 320 is configured to process the video data frame by frame. The display module 400 is configured to play the video data processed by the image processing unit 320.

The first illumination beam is configured as an illumination beam only containing wide-spectrum white visible light. The wavelength of the wide-spectrum white visible light is 400nm to 730nm.

The second illumination beam is configured as an illumination beam mixing a white visible light and a narrow-band IR excitation light. The wavelength of the narrow-band IR excitation light is 750nm to 810nm. The second illumination beam can interact with fluorescent substances contained in the object to form the reflected beams having the wavelength being 820nm or more and less than or equal to 860nm.

Preferably, the camera module 200 includes an optical adapter 230. The endoscope 210 is coupled to the operation portion 220 via the optical adapter 230. The optical adapter 230 is configured to focus the reflected beams.

The optical adapter 230 is formed with a cavity 231. At least one set of lenses 232 is arranged inside the cavity 231. The optical adapter 230 is configured to be rotatable about its own axis so that the lens 232 can move back and forth inside the lumen 231 so as to adjust the focal length of the fluorescence endoscope system and ensure its imaging clarity.

The endoscope 210 includes an objective lens 211, a light guide structure 212, an eyepiece 213, a light source interface 214, and a second light guide 215. The second light guide 215 is arranged axially along the endoscope 210. The first light guide of the light source module 100 is coupled to the second light guide via the light source interface 214. The second light guide 215 guides the illumination beams to irradiate the object. After the illumination beams encounter the object, the illumination beams change the propagation direction to form the reflected beams. The reflected beams enter the optical adapter 230 through the objective lens 211, the light guide structure 212 and the eyepiece 213..

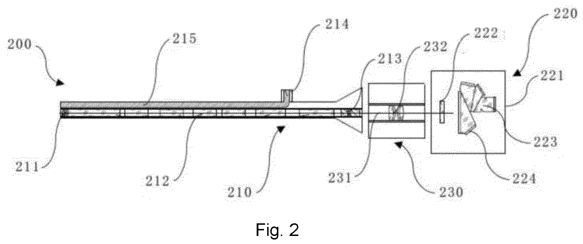

Referring to Fig. 2, the operation portion 220 includes a hand-held main body 221. The hand-held main body 221 is formed with a front end surface and a cavity. A light filter 222 is arranged on the front end surface. A beam-splitting prism 223 and an image sensor 224 are arranged inside the cavity. The adapter 230 focuses the reflected beams to the optical filter 222. The optical filter 222 is configured to cut off the reflected beams of a specific wavelength range. The beam-splitting prism 223 is configured to split the reflected beams passing through the optical filter 222. The image sensor 224 is configured to receive the reflected beams being split by the beam-splitting prism 223 and then convert the reflected beams into video data.

In the white-light mode, the light source module 100 emits the first illumination beam via the first light guide. The first illumination beam irradiates the object to form a first reflected beam. The optical adapter 230 focuses the first reflected beam to the optical filter 222. The optical filter 222 cuts off the first reflected beam of a specific wavelength range. The beam-splitting prism 223 splits the first reflected beam passing through the optical filter 222 into R/G/B-band reflected beam. The wavelength of the R/G/B-band reflected beam is 400nm to 730nm. The image sensor 224 receives the R/G/B-band reflected beam being split by the beam-splitting prism 223, and then converts the R/G/B-band reflected beam into R/G/B-channel video data. The image processing unit 320 performs frame-by-frame denoising, correction and gain processing on the R/G/B-channel video data to generate video data of white-light image. Finally, the display module 400 plays the video data of white-light image generated by the image processing unit 320.

In the fluorescence mode, the light source module 100 emits a second illuminating beam via the first light guide. The second illuminating beam irradiates the object to form a second reflected beam. The optical adapter 230 focuses the second reflected beam to the optical filter 222. The optical filter 222 cuts off the second reflected beam of a specific wavelength range. The beam-splitting prism 223 splits the second reflected beam passing through the optical filter 222 into R/G/B-band reflected beam and IR-band reflected beam. The wavelength of the R/G/B-band reflected beam is 400nm to 730nm. The wavelength of the IR-band reflected beam is 820nm to 860nm. The image sensor 224 receives the R/G/B-band reflected beam and the IR-band reflected beam, and converts the R/G/B-band reflected beam and the IR-band reflected beam into R/G/B-channel video data and IR-channel video data. The image processing unit 320 process the R/G/B-channel video data and IR-channel video data by denoising, correction and gain processing frame by frame to generate video data of white-light image and video data of fluorescence image, and process the video data of white-light image and the video data of fluorescence image by superimposing and mixing frame by frame to generate video data fusing white-light image and fluorescence image. The image processing unit 320 needs to combine the video data of white-light image, the video data of fluorescence image, and video data fusing white-light image and fluorescence image frame by frame to generate video data of multi-screen image including more than three images. Finally, the display module 400 plays the video data of white-light image, the video data of fluorescence image, the video data fusing white-light image and fluorescence image, and the video data of multi-screen image

Preferably, before a part of the narrow-band IR excitation light of the second illuminating beam reacts with the fluorescent substance contained in the object, the part of the narrow-band IR excitation light may change the propagation direction after irradiating the object to form the second reflected beam. In order to avoid the second reflected beam enters into the optical adapter 230, the optical filter 222 is configured to cut off the reflected beams with a wavelength greater than or equal to 750 nm and less than or equal to 810 nm, to enhance the fluorescence imaging effect.

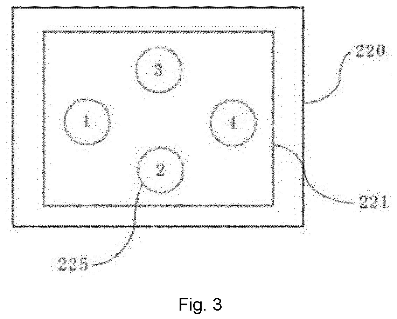

Referring to Fig. 3, the operation portion 220 also includes a first button 225 arranged on the handheld main body 221. The operation portion 220 is configured to generate a first control signal according to the first button 225 that is triggered, and transmit the first control signal to the image processing unit 320. The light source module 100 can be controlled by the first control signal to emit the first illumination beam or the second illumination beam, to switch between the white-light mode and the fluorescence mode.

Preferably, the operation portion 220 is configured to being preset with the first mapping relationship between the first button 225 and the first control signal, so that when the first button 225 is triggered, the first control signal is generated according to the first mapping relationship. The first mapping relationship includes a first mapping relationship a and a first mapping relationship b corresponding to the white-light mode and the fluorescence mode respectively, so as to control the image processing unit 320 to perform corresponding actions.

Preferably, as an alternative of the first button 225, the operation portion 220 is configured to being preset with the first virtual key code table of the first button 225. When the first button 225 is triggered, a first virtual key code is generated by matching the first virtual key code table. The first control signal is generated by the first virtual key code based on the first virtual key code table.

Specifically, the first button 225 includes a first physical button 1, a first physical button 2, a first physical button 3 and a first physical button 4.

In the white-light mode, the first virtual key code includes the first virtual key code a1 of the first physical button 1, the first virtual key code a2 of the first physical button 2, the first virtual key code a3 of the first physical button 3 and the first virtual key code a4 of the first physical button 4. The first control signal includes the first control signal a1, the first control signal a2, the first control signal a3, the first control signal a4, the first control signal a5 and the first control signal a6. After the first physical button 1, the first physical button 2, the first physical button 3 and the first physical button 4 are respectively triggered, the first virtual key code a1, the first virtual key code a2, the first virtual key code a3 and the first virtual key code a4 are generated firstly, and then the first control signal a1, the first control signal a2 or the first control signal a4, the first control signal a3 or the first control signal a5, and the first control signal a6 by the first virtual key code a1, the first virtual key code a2, the first virtual key code a3 and the first virtual key code a4 according to the first mapping relationship a. The first control signal a1, the first control signal a2 or the first control signal a4, the first control signal a3 or the first control signal a5, and the first control signal a6 are transmitted to the image processing unit 320 to control the image processing unit 320 to perform corresponding actions.

The first control signal a1 is generated after the first physical button 1 is triggered. The first control signal a1 is used to control the image processing unit 320 to scale up the video data of white-light image within a specific magnification range level by level. The first control signal a2 or first control signal a3 is generated after the first physical button 2 or the first physical button 3 is triggered an odd number of times. The first control signal a2 or first control signal a3 is used to control the image processing unit 320 to start modifying the video data of white-light image with specific technical means. The first physical button 2 or the first physical button 3 is triggered an even number of times, the first control signal a4 or first control signal a5 is generated to control the image processing unit 320 to stop modifying the video data of white-light image. After the first physical button 4 is triggered, the first control signal a6 is generated and transmitted to the control unit 310 via the image processing unit 320, to switch from the white-light mode to the fluorescence mode.

In the white-light mode, the image processing unit 320 is configured to scale up gradually the video data of white-light image at a magnification of 0.5 times within the magnification of 1 to 5 times. When the magnification is 5, the operation portion 220 transmits again the first control signal a1, to control the image processing unit 320 to reduce the video data of white-light image to be the magnification of 1. The specific technical means mentioned above are wide dynamic range, shadow correction, exposure correction, blood vessel enhancement or smoke removal. The first physical button 2 and the first physical button 3 correspondingly trigger different specific technical means.

In fluorescence mode, the first virtual key code includes the first virtual key code b1 of the first physical button 1, the first virtual key code b2 of the first physical button 2, the first virtual key code b3 of the first physical button 3 and the first virtual key code b4 of the first physical button 4. The first control signal includes the first control signal b1, the first control signal b2, the first control signal b3 and the first control signal b4. After the first physical button 2, the first physical button 3, and the first physical button 4 are respectively triggered, the first virtual key code b1, the first virtual key code b2, the first virtual key code b3 and the first virtual key code b4 are generated based on the first virtual key code table firstly, and then the first control signal b1, the first control signal b2, the first control signal b3 and the first control signal b4 by the first virtual key code b1, the first virtual key code b2, the first virtual key code b3 and the first virtual key code b4 based on the first mapping relationship b. the first control signal b1, the first control signal b2, the first control signal b3 and the first control signal b4 are transmitted to the image processing unit 320, to control the image processing unit 320 to perform corresponding actions.

After the first physical button 1 is triggered, the first control signal b1 is generated to control the image processing unit 320 to sequentially and cyclically transmit the video data of white-light image, the video data of fluorescence image, the video data fusing white-light image and fluorescence image, or the video data of multi-screen image to the display module 400. After the first physical button 2 or the first physical button 3 is triggered, the first control signal b2 or the first control signal b3 is generated and transmitted to the control unit 310 via the image processing unit 320. The first control signal b2 or the first control signal b3 is used to control the light source module 100 to adjust its own supply current within a specific range to enhance or weaken the brightness of the narrow-band IR excitation light of the second illumination beam. After the first physical button 4 is triggered, the first control signal b4 is generated and transmitted to the control unit 310 via the image processing unit 320 , and is used to switch from the fluorescence mode to the white-light mode.

In the fluorescence mode, the light source module 100 is configured to adjust its own supply current within the range of 0.5A to 18A.

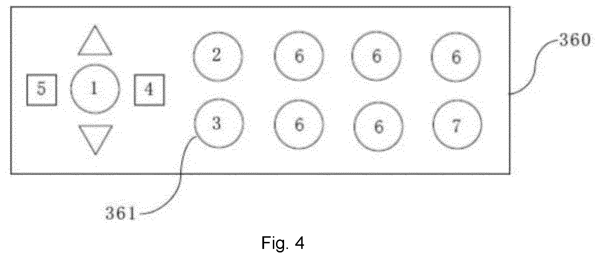

Referring to Fig. 4, the main control module 300 includes a panel assembly 360 and a second button 361. The second button 361 is arranged on the panel assembly 360. The panel assembly 360 is configured to generate a second control signal according to the triggered second button 361, and transmit the second control signal to the control unit 310. The second control signal can control the light source module 100 to emit the first illumination beam or the second illumination beam to switch between the white-light mode and the fluorescence mode.

Preferably, the panel assembly 360 is configured to being preset with a second mapping relationship between the second button 361 and the second control signal, so that when the second button 361 is triggered, the second control signal is generated according to the second mapping relationship to drive the image processing unit 320 to perform corresponding actions.

Specifically, the second button 361 includes a second physical button 1, a second physical button 2, a second physical button 3, a second physical button 4, a second physical button 5, a second physical button 6 and a second physical button 7. The second control signal includes a second control signal 1, a second control signal 2, a second control signal 3, a second control signal 4, a second control signal 5, a second control signal 6, a second control signal 7, a second control signal 8, a second control signal 9, a second control signal 10, a second control signal 11, and a second control signal 12. The second physical button 1, the second physical button 2, the second physical button 3, the second physical button 4, the second physical button 5, the second physical button 6, and the second physical button 7 are respectively triggered, the second control signal 1 or the second control signal 2, the second control signal 3 or the second control signal 4, the second control signal 5 or the second control signal 6, the second control signal 7, the second control signal 8, the second control signal 9 or the second control signal 10, and the second control signal 11 or the second control signal 12 are generated according to the second mapping relationship. The second control signal 1 or the second control signal 2, the second control signal 3 or the second control signal 4, the second control signal 5 or the second control signal 6, the second control signal 7, the second control signal 8, the second control signal 9 or the second control signal 10, and the second control signal 11 or the second control signal 12 are transmitted to the control unit 310.

In order to debug the fluorescence endoscope system in the absence of external devices, the image processing unit 320 is configured to load a first GUI (Graphical User Interface) for setting the parameters of the main control module 300. The first GUI is overlapped and displayed on the upper layer of the video data played by the display module 400. In the white-light mode or the fluorescence mode, after the second physical button 1 is triggered an odd number of times, the panel assembly 360 generates the second control signal 1, and transmits the second control signal 1 to the control unit 310. The control unit 310 transmits the second control signal 1 to the image processing unit 320 to control the image processing unit 320 to load the first GUI. After the second button 1 is triggered an even number of times, the panel assembly 360 generates the second control signal 2, and transmits the second control signal 2 to the control unit 310. The control unit 310 transmits the second control signal 2 to the image processing unit 320 to control the image processing unit 320 to remove the first GUI.

The image processing unit 320 is configured to load a second GUI for brightening or darkening the video data of white-light image, the video data of fluorescence image, the video data fusing white-light image and fluorescence image or the video data of multi-screen image. The second GUI is overlapped and displayed on the upper layer of the video data played by the display module 400. In the white-light mode or the fluorescence mode, after the second physical button 2 is triggered an odd number of times, the panel assembly 360 generates a second control signal 3, and transmits the second control signal 3 to the control unit 310. The control unit 310 transmits the second control signal 3 to the image processing unit 320 to control the image processing unit 320 to load the second GUI. After the second physical button 2 is triggered an even number of times, the panel assembly 360 generates the second control signal 4 and transmits the second control signal 4 to the control unit 310. The control unit 310 transmits the second control signal 4 to the image processing unit 320 to control the image processing unit 320 to remove the second GUI.

In the white-light mode, the second GUI is overlapped and displayed on the upper layer of the video data played by the display module 400. After the second physical button 4 or the second physical button 5 is triggered every single time, the panel assembly 360 generates the second control signal 7 or the second control signal 8, and transmits the second control signal 7 or the second control signal 8 to the control unit 310. The control unit 310 transmits the second control signal 7 or the second control signal 8 to the image processing unit 320, to control the image processing unit 320 to brighten or darken the video data of white-light image.

In the fluorescence mode, the second GUI is overlapped and displayed on the upper layer of the video data played by the display module 400. After the second physical button 4 or the second physical button 5 is triggered every single time, the panel assembly 360 generates the second control signal 7 or the second control signal 8, and transmits the second control signal 7 or the second control signal 8 to the control unit 310. The control unit 310 transmits the second control signal 7 or the second control signal 8 to the image processing unit 320, to control the image processing unit 320 to brighten and darken the video data of white-light image, the video data of fluorescence image, the video data fusing white-light image and fluorescence image, or the video data of multi-screen image.

The image processing unit 320 is configured to load a third GUI for scaling the video data of white-light image, the video data of fluorescence image, the video data fusing white-light image and fluorescence image, or the video data of multi-screen image within a specific magnification range level by level. The third GUI is overlapped and displayed on the upper layer of the video data played by the display module 400. In the white-light mode or the fluorescence mode, after the second physical button 3 is triggered an odd number of times, the panel assembly 360 generates the second control signal 5, and transmits the second control signal 5 to the control unit 310. The control unit 310 transmits the second control signal 5 to the image processing unit 320 to control the image processing unit 320 to load the third GUI. After the second physical button 3 is triggered an even number of times, the panel assembly 360 generates the second control signal 6 and transmits the second control signal 6 to the control unit 310. The control unit 310 transmits the second control signal 6 to the image processing unit 320 to control the image processing unit 320 to remove the third GUI.

The third GUI is configured to scale gradually the video data of white-light image, the video data of fluorescence image, the video data fusing white-light image and fluorescence image or the video data of multi-screen image at a magnification of 0.5 times within a magnification range of 1 to 5 times.

In the white-light mode, the third GUI is overlapped and displayed on the upper layer of the video data played by the display module 400. After the second physical button 4 or the second physical button 5 is triggered every single time, the panel assembly 360 generates the second control signal 7 or the second control signal 8, and transmits the second control signal 7 or the second control signal 8 to the control unit 310. The control unit 310 transmits the second control signal 7 or the second control signal 8 to the image processing unit 320, to control the image processing unit 320 to scale up or scale down the video data of white-light image.

In the fluorescence mode, the third GUI is overlapped and displayed on the upper layer of the video data played by the display module 400. After the second physical button 4 or the second physical button 5 is triggered every single time, the panel assembly 360 generates the second control signal 7 or the second control signal 8, and transmits the second control signal 7 or the second control signal 8 to the control unit 310. The control unit 310 transmits the second control signal 7 or the second control signal 8 to the image processing unit 320, to control the image processing unit 320 to scale up or scale down the video data of white-light image, the video data of fluorescence image, the video data fusing white-light image and fluorescence image, or the video data of multi-screen image.

In the white-light mode, after the second physical button 6 is triggered an odd number of times, the panel assembly 360 generates the second control signal 9 correspondingly, and transmits the second control signal 9 to the control unit 310.The control unit 310 transmits the second control signal 9 to the image processing unit 320 to start modifying the video data of white-light image with specific technical means. After the second button 6 is triggered an even number of times, the panel assembly 360 generates the second control signal 10, and transmits the second control signal 10 to the control unit 310. The control unit 310 transmits the second control signal 10 to the image processing unit 320, to control the image processing unit 320 to stop modifying the video data of white-light image.

In the fluorescence mode, after the second physical button 6 is triggered an odd number of times, the panel assembly 360 generates a second control signal 9, and transmits the second control signal 9 to the control unit 310. The control unit 310 transmits the second control signal 9 to the image processing unit 320 to control the image processing unit 320 to modify the video data of white-light image, video data of fluorescence image, video data fusing white-light image and fluorescence image or video data of multi-screen image by specific technical means. After the second physical button 6 is triggered an even number of times, the panel assembly 360 generates the second control signal 10, and transmits the second control signal 10 to the control unit 310. The control unit 310 transmits the second control signal 10 to the image processing unit 320 to control the processing unit 320 to stop modifying the video data of white-light image, the video data of fluorescence image, the video data fusing white-light image and fluorescence image, or the video data of multi-screen image.

The above-mentioned specific technical means are wide dynamic range, shadow correction, exposure correction, blood vessel enhancement or smoke removal. The second physical button 6 correspondingly triggers different specific technical means.

In the white-light mode, after the second physical button 7 is triggered, the panel assembly 360 correspondingly generates the second control signal 11, and transmits the second control signal 11 to the control unit 310. The control unit 310 controls the light source module 100 to emit the second illumination beam to switch the fluorescence mode. In the fluorescence mode, after the second physical button 7 is triggered, the panel assembly 360 generates the second control signal 12 correspondingly, and transmits the second control signal 12 to the control unit 310. The control unit 310 controls the light source module 100 to emit the first illumination beam to switch the white-light mode. Unlike the way that the operation portion 220 transmits the first control signal a6 or the first control signal b4 to the control unit 310 via the image processing unit 320, the panel assembly 360 directly transmits the second control signal 11 or the second control signal 12 to the control unit 310, which is different from. By this way, when the first physical button 4 and the second physical button 7 are triggered simultaneously, the second control signal 11 or the second control signal 12 will reach at the control unit 310 before the first control signal a6 or the first control signal b4, thereby avoiding the signal conflict affects the imaging of fluorescence endoscopy system, and improving the safety of surgery.

Preferably, in order to prevent the parameters of the main control module 300 from being altering unintentionally, the panel assembly 360 is preset with the combination arrangement rule for triggering the second button, and the third mapping relationship between the combination arrangement rule and the second control signal. When the second button 361 is triggered according to the combination arrangement rule, the second control signal is generated according to the third mapping relationship. The second control signal is used to control the image processing unit 320 to load the first GUI on the upper layer of the video data.

Specifically, the second control signal includes the second control signal 13. In the white-light mode or the fluorescence mode, after the second physical button 2 and the second physical button 3 are triggered one time simultaneously and then the second physical button 1 is triggered one time, the panel assembly 360 correspondingly generates the second control signal 13 according to the combination arrangement rule and the third mapping relationship. The second control signal 13 is transmitted to the control unit 310. The second control signal 13 is transmitted by the control unit 310 to the image processing unit 320 to control the image processing unit 320 to load the first GUI.

As an alternative implementation, in the white-light mode or the fluorescence mode, after the second physical button 6 is triggered several times and then the second physical button 1 is triggered one time, the panel assembly 360 generates the second control signal 13 correspondingly. The second control signal 13 is transmitted to the control unit 310. The control unit 310 transmits the second control signal 13 to image processing unit 320 to control the image processing unit 320 to load the first GUI.

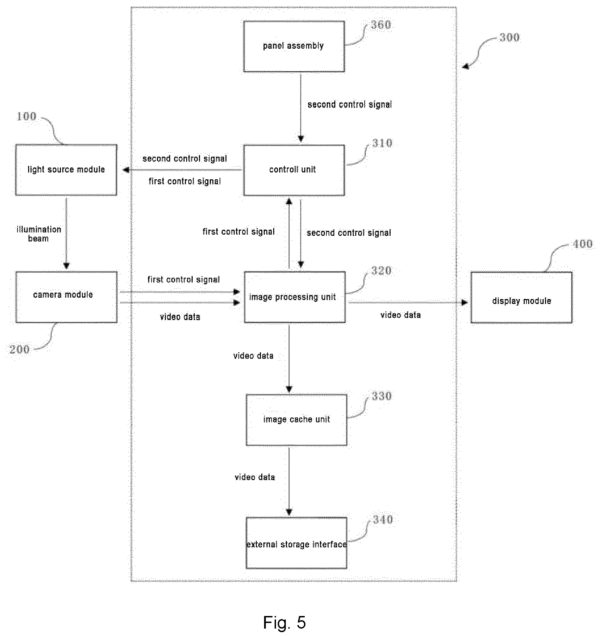

Preferably, referring to Fig. 5, the main control module 300 also includes an image cache unit 330 and an external storage interface 340. The image cache unit 330 is configured to record the video data processed by the image processing unit 320. The image cache unit 330 communicates with a storage device outside the main control module 300 through the external storage interface 340.

The present disclosure can provide at least two modes including the white-light mode and the fluorescence mode, which can be switched by the first button 225 on the operation portion 220. The present disclosure can provide the fluorescence images of a tissue under the tissue surface through the fluorescence imaging, so that the inconspicuous lesions and early lesions can be visualized and accurately identified, thereby improving the safety of surgery.

### EMBODIMENT TWO

The present disclosure also provides a method for controlling a fluorescence endoscope system. The method comprises:
triggering the first button 225 to control the light source module 100 to emit the first illumination beam or the second illumination beam to switch between the white-light mode and the fluorescence mode; and/or
triggering the second button 361 to control the light source module 100 to emit the first illumination beam or second illumination beam to switch between the white-light mode and the fluorescence mode.

Preferably, the control method of the present disclosure also comprises:
triggering the second button 361 according to the combination arrangement rule to control the image processing unit 320 to load the first GUI on the upper layer of the video data.

Preferably, the control method of the present disclosure also comprises:
rotating the optical adapter 230 to move the lens 232 back and forth inside the cavity 231 to adjust the focal length of the fluorescence endoscope system and ensure its imaging clarity.

The specific contents above have been described in detail in the embodiment one, and thus the present disclosure will not repeat the specific contents.

Obviously, the aforementioned embodiments are merely illustrative examples made for clarity and are not intended to limit the present disclosure. For those skilled in the art, other forms of variation or modification may be made based on the foregoing description. It is neither necessary nor possible to exhaustively enumerate all embodiments here. The obvious variations or modifications derived therefrom remain within the scope of protection of the present invention.

## Claims

1. A fluorescence endoscope system, comprising:
a light source module having a first light guide configured to guide illumination beams emitted by the light source module, wherein the illumination beams comprise a first illumination beam corresponding to a white-light mode and a second illumination beam corresponding to a fluorescence mode;
a camera module having an endoscope and an operation portion which are coupled to each other, wherein the endoscope is configured to propagate the illumination beams guided by the first light guide and reflected beams formed after the illumination beams are irradiated to an object, and the operation portion is configured to receive the reflected beams and convert the reflected beams into video data;
a main control module having a control unit and an image processing unit, wherein the control unit is configured to control the light source module to emit the first illumination beam or the second illumination beam, and the image processing unit is configured to process the video data; and
a display module configured to play the video data processed by the image processing unit,
wherein the operation portion comprises a hand-held main body and a first button arranged on the hand-held main body, the operation portion is configured to generate a first control signal after the first button is triggered, the operation portion transmits the first control signal to the image processing unit, and the first control signal is capable of controlling the light source module to emit the first illumination beam or the second illumination beam to switch between the white-light mode and the fluorescence mode.

2. The system of claim 1, wherein the first illumination beam is an illumination beam containing only a wide-spectrum white visible light having a wavelength of 400nm to 730nm, and the second illumination beam is an illumination beam mixing a white visible light and a narrow-band IR excitation light having a wavelength of 750nm to 810nm.

3. The system of claim 1, wherein the camera module comprises an optical adapter through which the endoscope is coupled to the operation portion, and the optical adapter is configured to focus the reflected beams.

4. The system of claim 3, wherein the endoscope comprises an objective lens, a light guide structure, an eyepiece, a light source interface, and a second light guide, the second light guide is arranged along the axial direction of the endoscope, the first light guide is coupled to the second light guide through the light source interface, the illumination beams encounter the object and change a propagation direction to form the reflected beams, and the reflected beams pass through the objective lens, the light guide structure, the eyepiece and enter into the optical adapter.

5. The system of claim 3, wherein the hand-held main body has a front end surface and a cavity, the front end surface is provided with an optical filter, a beam-splitting prism and an image sensor are arranged inside of the cavity, the optical adapter focuses the reflected beams on the optical filter, the optical filter is configured to cut off the reflected beams with a wavelength greater than or equal to 750nm and less than or equal to 81 0nm, the beam-splitting prism is configured to split the reflected beams passing through the optical filter, and the image sensor is configured to receive the reflected beams split by the beam-splitting prism and convert the reflected beams to the video data.

6. The system of claim 1, wherein the operation portion is preset with a first mapping relationship between the first button and the first control signal, the first control signal is generated based on the first mapping relationship after the first button is triggered, and the first control signal is used to control the image processing unit to perform corresponding actions, wherein the first mapping relationship includes a first mapping relationship a and a first mapping relationship b corresponding to the white-light mode and the fluorescence mode respectively.

7. The system of claim 1, wherein the main control module comprises a panel assembly and a second button arranged on the panel assembly, the panel assembly is configured to generate a second control signal after the second button is triggered, and the panel assembly transmits the second control signal to the control unit, wherein the second control signal is used to control the light source module to emit the first illumination beam or the second illumination beam to switch between the white-light mode and the fluorescence mode.

8. The system of claim 7, wherein the panel assembly is preset with the combination arrangement rule that the second button is triggered, and is preset with a third mapping relationship between the combination arrangement rule and the second control signal, and after the second button is triggered according to the combination arrangement rule, the second control signal is generated based on the third mapping relationship to control the image processing unit to load a first GUI on a upper layer of the video data.

9. A method for controlling the system of claim 8, comprising:
triggering the first button to control the light source module to emit the first illumination beam or the second illumination beam to switch between the white-light mode and the fluorescence mode; and/or
triggering the second button to control the light source module to emit the first illumination beam or the second illumination beam to switch between the white-light mode and the fluorescence mode.

10. A method of claim 9, further comprising: triggering the second button according to the combination arrangement rule, to control the image processing unit to load the first GUI on the upper layer of the video data.
